(19) Europäisches Patentamt
European Patent Office
Office européen des brevets

(11) EP 0 637 299 B1

# (12) EUROPÄISCHE PATENTSCHRIFT

(45) Veröffentlichungstag und Bekanntmachung des Hinweises auf die Patenterteilung:
**22.05.1996 Patentblatt 1996/21**

(21) Anmeldenummer: **92924724.5**

(22) Anmeldetag: **14.12.1992**

(51) Int Cl.[6]: **C07C 401/00**, A61K 31/59

(86) Internationale Anmeldenummer:
**PCT/EP92/02887**

(87) Internationale Veröffentlichungsnummer:
**WO 93/12081 (24.06.1993 Gazette 1993/15)**

## (54) 20-METHYL-SUBSTITUIERTE VITAMIN D-DERIVATE

20-METHYL-SUBSTITUTED VITAMIN D DERIVATES

DERIVES DE VITAMINE D A SUBSTITUTION 20-METHYLE

(84) Benannte Vertragsstaaten:
**AT BE CH DE DK ES FR GB GR IE IT LI LU MC NL PT SE**

(30) Priorität: **13.12.1991 DE 4141746**

(43) Veröffentlichungstag der Anmeldung:
**08.02.1995 Patentblatt 1995/06**

(73) Patentinhaber: **SCHERING AKTIENGESELLSCHAFT**
**D-13353 Berlin (DE)**

(72) Erfinder:
- **NEEF, Günter**
  **D-1000 Berlin 31 (DE)**
- **STEINMEYER, Andreas**
  **D-1000 Berlin 19 (DE)**
- **KIRSCH, Gerald**
  **D-1000 Berlin 33 (DE)**
- **SCHWARZ, Katica**
  **D-1000 Berlin 10 (DE)**
- **THIEROFF-Ekerdt, Ruth**
  **D-1000 Berlin 28 (DE)**
- **WIESINGER, Herbert**
  **D-1000 Berlin 30 (DE)**
- **HABEREY, Martin**
  **D-1000 Berlin 46 (DE)**

(56) Entgegenhaltungen:
- **KIDNEY INTERNATIONAL SUPPLEMENT Bd. 38, Nr. 29, 1990, NEW YORK US Seiten S22 - S27 A. J. BROWN ET AL 'New Active Analogues of Vitamin D3 with Low Calcemic Activity'**
- **JOURNAL OF BIOLOGICAL CHEMISTRY Bd. 254, Nr. 22, 25. November 1979, BALTIMORE MD US Seiten 11445 - 11449 A. W. NORMAN ET AL 'Structure Function Studies of the Side Chain of 25-Hydroxyvitamin D3'**
- **JOURNAL OF MEDICINAL CHEMISTRY Bd. 20, Nr. 1, Januar 1977, WASHINGTON US Seiten 5 - 11 R. L. JOHNSON ET AL 'Studies on Vitamin D3 (Calciferol) and its Analogues. 10. Side Chain Analogues of 25-Hydroxyvitamin D3'**
- **Nature 353,297 (1991)Vol.88.pp 3060-3064,April 1991**
- **Int.J.Derm,31,113 FF (1992)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 637 299 B1

## Beschreibung

Die vorliegende Erfindung betrifft 20-Methyl-substituierte Vitamin D-Derivate der allgemeinen Formel I

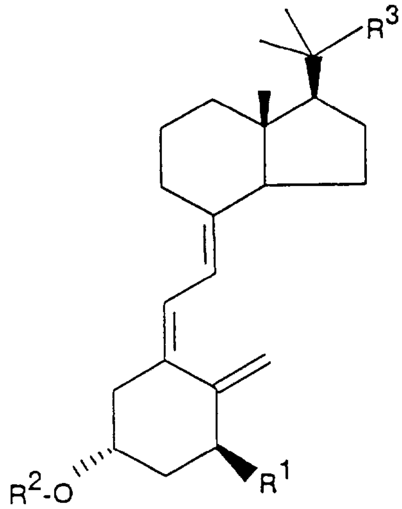

(I),

worin

$R^1$ ein Wasserstoffatom, eine Hydroxygruppe oder eine Alkanoyloxygruppe mit 1 bis 12 Kohlenstoffatomen bzw. eine Benzoyloxygruppe,
$R^2$ ein Wasserstoffatom oder eine Alkanoylgruppe mit 1 bis 12 Kohlenstoffatomen bzw. eine Benzoylgruppe und
$R^3$ einen gesättigten oder ungesättigten, geradkettigen oder verzweigten Kohlenwasserstoffrest mit bis zu 18 C-Atomen, der gegebenenfalls durch eine oder mehrere $C_{3-10}$-Cycloalkyl- oder Cycloalkenylrest(e), letztere mit bis zu 2 Doppelbindungen unterbrochen und/oder substituiert ist, der gegebenenfalls mit einer oder mehreren Hydroxy-, Oxo-, Aminogruppe(n) und/oder einem oder mehreren Halogenatom(en) substituiert sowie gegebenenfalls ein oder mehrere Sauerstoff-, Schwefel- und/oder >NH-Gruppe(n) als verbrückende(s) Kettenglied(er) im Kohlenwasserstoffrest aufweist,
bedeuten,
ein Verfahren zu deren Herstellung, pharmazeutische Präparate, die diese Verbindungen enthalten sowie deren Verwendung zur Herstellung von Arzneimitteln.

Die bevorzugten Reste $R^1$ und $R^2$ leiten sich von $C_1$- bis $C_9$-, insbesondere $C_2$- bis $C_5$- Alkancarbonsäuren ab, wie beispielsweise Acetyl(oxy)-, Propionyl(oxy)-, Butyryl(oxy)-.

Bevorzugte Reste $R^3$ sind die nachfolgend strukturformelmäßig genannten Ketten:

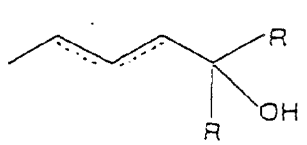

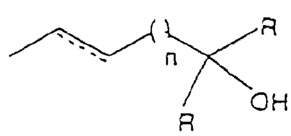

n = 1 - 3

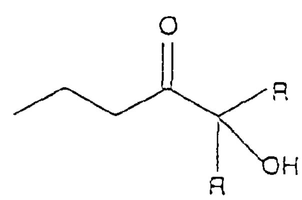

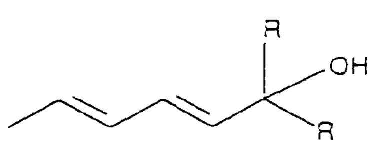

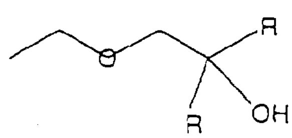

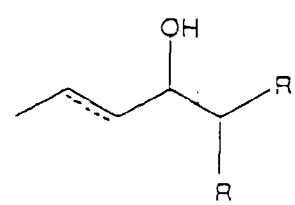

R = $C_1$-$C_4$-Alkyl, -Hydroxyalkyl, -O-Alkyl.

Insbesondere sind folgende erfindungsgemäße Verbindungen zu nennen:

1α,25-Dihydroxy-20,26,27-trimethyl-23-oxa-vitamin $D_3$,
1(S),3(R)-Dihydroxy-20-(5-hydroxy-5-methyl-hexa-1E,3E-dien-1-yl)-20-methyl-9,10-secopregna-5Z,7E,10(19)-trien,
1α,25-Dihydroxy-20-methyl-vitamin $D_3$,
1α,25-Dihydroxy-20-methyl-24-homo-vitamin $D_3$,
1α,24(S)-Dihydroxy-20-methyl-vitamin $D_3$,
1α,25-Dihydroxy-20-methyl-23-oxa-vitamin $D_3$,
1α,24(R),25-Trihydroxy-20-methyl-vitamin $D_3$,
1α,24(S),25-Trihydroxy-20-methyl-vitamin $D_3$,
1α,25-Dihydroxy-20-methyl-24-oxo-vitamin $D_3$,
(5Z,7E)-(1S,3R)-20-Methyl-20-vinyl-9,10-seco-pregna-5,7,10(19)-trien-1,3-diol,
(5Z,7E)-(1S,3R)-20-Ethyl-20-methyl-9,10-seco-pregna-5,7,10(19)-trien-1,3-diol,
(5Z,7E)-(1S,3R)-20-Hydroxymethyl-20-methyl-9,10-seco-pregna-5,7,10(19)-trien-1,3-diol,
1α,25-Dihydroxy-20-methyl-23,24-dehydro-vitamin $D_3$,
1α,25-Dihydroxy-20,26,27-trimethyl-23,24-dehydro-vitamin $D_3$.

Die natürlichen Vitamine $D_2$ und $D_3$ (vergl. allgemeine Formel VI) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$- und Plasma-Phosphat-Spiegels; sie wirken einem Absinken des Plasma-$Ca^{++}$-Spiegels entgegen.

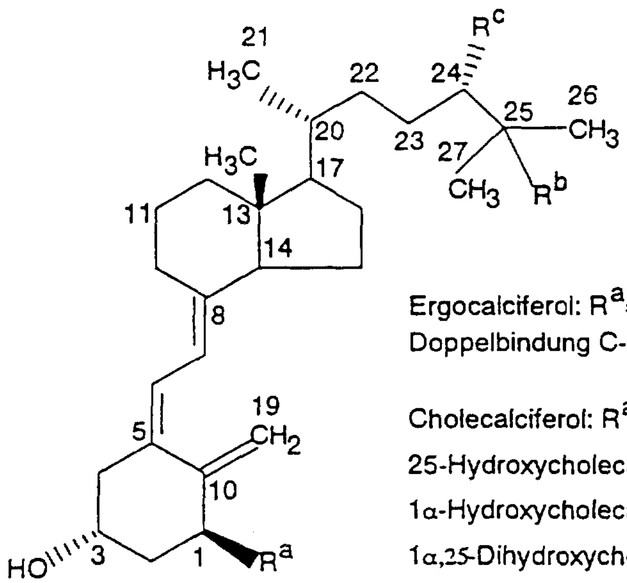

(VI)

Ergocalciferol: $R^a=R^b=H$, $R^c=CH_3$ Doppelbindung C-22/23 — Vitamin D2

Cholecalciferol: $R^a=R^b=R^c=H$ — Vitamin D3

25-Hydroxycholecalciferol: $R^a=R^c=H, R^b=OH$

1α-Hydroxycholecalciferol: $R^a=OH, R^b=R^c=H$

1α,25-Dihydroxycholecalciferol: $R^a=R^b=OH, R^c=H$ — Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, "The Metabolism and Function of Vitamin D" in Biochemistry of Steroid Hormones, Hrsg. H.L.J. Makin, 2nd Edition, Blackwell Scientific Publications 1984, S. 71-116).

Bei Vitamin D-Anwendung kann es aber zu Überdosierungserscheinungen kommen (Hypercalcämie).

In 24-Stellung hydroxylierte 1α-Cholecalciferole gehen bereits aus der DE-AS-25 26 981 hervor; sie besitzen eine geringere Toxizität ais das entsprechende nicht-hydroxyiierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol.

Die in der internationalen Patentanmeldung WO 87/00834 beschriebenen 24-Hydroxy-Vitamin D-analoga können für die Behandlung von durch abnormer Zellproliferation und/oder Zelldifferentiation hervorgerufenen Störungen beim Menschen und Tier dienen.

Für verschiedene 1,25-Dihydroxy-Homo-Vitamin D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Die neuen Vitamin D-Derivate der allgemeinen Formel I zeichnen sich gegenüber den bereits bekannten seitenketten-modifizierten Verbindungen mit Vitamin D-Aktivität durch eine zusätzliche Methylgruppe am Kohlenstoffatom 20 aus. Auf diese Weise verliert die Position C-20 den Charakter eines Asymmetriezentrums.

Neben den dadurch bedingten Vereinfachungen bei Synthese und Aufreinigung von Zwischen- und Endprodukten entstehen so neue Verbindungen, die überraschend hohe biologische Wirksamkeit aufweisen. Gemessen am Standard Calcitriol (1α,25-Dihydroxyvitamin $D_3$), zeigen die erfindungsgemäßen Substanzen bei vergleichbarer Affinität zum Calcitriolrezeptor eine um mehrere Zehnerpotenzen verbesserte Induktion der Zelldifferenzierung (HL-60), lösen die Aufagbe der Bereitstellung von Arzneimitteln, die alternative Zellproliferationshemmer sind, und eignen sich daher in besonderer Weise zur Behandlung von Erkrankungen, die durch Hyperprolifertion und gestörte Zelldifferenzierung gekennzeichnet sind wie z.B. hyperproliferative Erkrankungen der Haut (Psoriasis), maligne Tumoren (Leukämie, Coloncarcinom, Mammacarcinom) und Akne (J. Invest. Dermatol. Vol. 92 No. 3, 1989). In einer besonders bevorzugten Ausführungsform der Erfindung werden vor der Behandlung im Zielorgan Calcitriolrezeptoren nachgewiesen.
Daneben können die neuen Verbindungen natürlich auch in ähnlicher Weise wie die bekannten Vitamin D-Abkömmlinge zur Behandlung von Störungen des Calciumstoffwechsels, zur Immunmodulation und zur Verlangsamung der Hautalterung verwendet werden.

Die Vitamin D-Aktivität der erfindungsgemäßen Verbindungen wird mittels des Calcitriol-Rezeptortests bestimmt. Er wird unter Verwendung eines spezifischen Rezeptorproteins aus dem Darm von jungen Schweinen durchgeführt. Rezeptorhaltiges Bindungsprotein wird mit $^3$H-Calcitriol ($5x10^{-10}$ mol/l) in einem Reaktionsvolumen von 0,270 ml in Abwesenheit und in Anwesenheit der Prüfsubstanzen für 2 Stunden bei 4°C in einem Teströhrchen inkubiert. Zur Trennung von freiem und rezeptorgebundenem Calcitriol wird eine Charcoal-Dextran-Absorption durchgeführt. Dazu werden 250 μl einer Charcoal-Dextran-Suspension jedem Teströhrchen zugeführt und bei 4°C für 20 Minuten inkubiert. Anschließend werden die Proben bei 10 000 x g 5 Minuten bei 4°C zentrifugiert. Der Überstand wird dekantiert und nach Istündiger Äquilibrierung in Picofluor 15 TM in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzen
1(S),(3R)-Dihydroxy-20-(5-hydroxy-5-methyl-hexa-1E,3E-dien-1-yl)-20-methyl-9,10-secopregna-5Z,7E,10(19)-trien, 1α,25-Dihydroxy-20,26,27-trimethyl-23-oxa-vitamin $D_3$ und 1α,25-Dihydroxy-20-methyl-23,24-dehydro-vitamin $D_3$ KF-Werte von 3,4, 1,6 bzw. 0,8.

Die stark verbesserte Induktion der Zelldifferenzierung der neuen Verbindungen ergibt sich aus dem nachstehend beschriebenen Test.
Es ist literaturbekannt (Mangelsdorf, D.J. et al., J. Cell. Biol. 98: 391-398 (1984)), daß die Behandlung humaner Leukämiezellen (Promyelozytenzellinie HL 60) in vitro mit Calcitriol die Differenzierung der Zellen zu Makrophagen induziert.

HL 60-Zellen werden in Gewebekulturmedium (RPMI - 10% fetales Kälberserum) bei 37°C in einer Atmosphäre 5% $CO_2$ in Luft kultiviert.

Zur Substanztestung werden die Zellen abzentrifugiert und 2.8 x $10^5$ Zellen/ml in. phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 μl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5% $CO_2$ in Luft wird in jede Vertiefung der 96-Loch-Platte zu der Zellsuspension 100 μl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^{-7}$ mol/l) pipettiert.

Durch Inkubation über 2 Stunden bei 37°C und 5% $CO_2$ in Luft wird infolge der intrazellulären Sauerstoffradikalfreisetzung ($O_2^{2-}$), stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die anhaftenden Zellen durch Zugabe von Methanol fixiert und nach Fixation getrocknet.

Zur Lösung der gebildeten intrazellulären Formazankristalle werden in jede Vertiefung 100 μl Kaliumhydroxid (2 mol/l) und 100 μl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem Formazan. Die relative Wirksamkeit der Testsubstanz ergibt sich aus dem Quotienten $ED_{50}$ Testsubstanz/$ED_{50}$ Calcitriol. Die erfindungsgemäßen Verbindungen erweisen sich dabei als um mehrere Zehnerpotenzen wirksamer als Calcitriol

Die immunmodulatorische Wirkung der erfindungsgemäßen Verbindungen ergibt sich aus der Inhibierung der Proliferation stimulierter humaner Lymphocyten und deren Interleukin 2 (IL 2) Produktion.

Es wurde nun gefunden, daß die erfindungsgemäßen Verbindungen potente Hemmstoffe der Proliferation und Interleukin 2 (IL 2)-Synthese von humanen Lymphocyten sind.

Zur Bestimmung der Lymphocytenproliferation wurden mononukleäre Zellen aus Citratblut durch Dichtegradientenzentrifugation gewonnen und $5x10^4$ Zellen/200 µl in Mikrotiterplatten in 200 µl Gewebekulturmedium (RPMI 1640 unter Zusatz von 10% fetalem Kälberserum) kultiviert. Bei der Aussaat wurden Phythämagglutinin (PHA) (5 µg) und unterschiedliche Konzentrationen der Testsubstanz oder Calcitriol (1,25-Dihydroxycholecalciferol) als Standard zugegeben und die Zellen für 96 Stunden bei 37°C in einer Atmosphäre von 5% $CO_2$ in Luft inkubiert. Für die letzten 6 Stunden wurde den Zellen 0,2 µCi/Loch [$^3$H]-Thymidin zugesetzt.
Danach wurden die Zellen über einen Glasfaserfilter abgesaugt und die Radioaktivität der Filter als Maß für den Einbau von [$^3$H]-Thymidin und damit für die Zellproliferation im β-Counter gemessen.

Zur Bestimmung der IL 2-Sekretion wurden mononukleäre Zellen aus humanem Blut wie zur Bestimmung der Proliferation präpariert und $2x10^6$ Zellen in 1 ml Gewebekulturmedium (RPMI 1640 unter Zusatz von 2% fetalem Kälberserum) in 24-Loch-Platten kultiviert. Bei der Aussaat wurden PHA (20 µg) und unterschiedliche Konzentrationen der Testsubstanz oder Calcitriol als Standard zugegeben. 24 Stunden nach Kultur bei 37°C in einer Atmosphäre von 5% $CO_2$ in Luft wurde der Gewebekulturüberstand durch Zentrifugation gewonnen und im Überstand die IL 2 Konzentration mit einem ELISA quantifiziert.

Die Lymphocytenproliferation wurde um 50% durch 1α,25-Dihydroxy-20-methyl-23,24-dehydro-vitamin $D_3$ in einer Konzentration von $1x10^{-11}$, und durch Calcitriol in einer Konzentration von $4x10^{-10}$ um 50% gehemmt.

Die IL 2-Sekretion wurde durch 1α,25-Dihydroxy-20-methyl-23,24-dehydro-vitamin $D_3$ im gleichen Konzentrationsbereich gehemmt.

Infolge der Hemmung der Lymphocytenproliferation und IL 2-Synthese in niedriger Konzentration sind die erfindungsgemäßen Verbindungen der allgemeinen Formel I zur Therapie von Erkrankungen des Immunsystems geeignet, z.B. Erkrankungen des atopischen Formenkreises (atopische Dermatitis, Asthma), Autoimmunerkrankungen einschließlich Diabetes mellitus, Transplantatabstoßungsreaktionen und AIDS.

Für Calcitriol wurde gefunden, daß es aufgrund eines rezeptorvermittelten Mechanismus nicht nur die IL 2-Sekretion, sondern auch die Produktion anderer entzündungsfördernder Cytokine hemmt. Da die Verbindungen der allgemeinen Formel I etwa ebenso gut an den Rezeptor binden wie Calcitriol, sind sie geeignet zur Behandlung von entzündlichen Erkrankungen wie Arthritis, Colitis ulcerosa und Morbus Crohn.

Bei der Behandlung von Autoimmunerkrankungen, Transplantatabstoßungsreaktionen und AIDS können die neuen Verbindungen der allgemeinen Formel I vorteilhaft mit anderen immunsuppressiv wirksamen Stoffen wie Cyclosporin A und FK 506 kombiniert werden.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten. Die Verbindungen können formuliert werden als Lösungen in pharmazeutisch verträglichen Solventien oder als Emulsionen, Suspensionen oder Dispersionen in geeigneten pharmazeutischen Solventien oder Trägem oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotions oder geeigneter transdermaler Pflaster appliziert, wie in der EP-A-0 387 077 beschrieben ist.

Die tägliche Dosis liegt bei

0,1 µg/Patient/Tag - 1000 µg (1 mg)/Patient/Tag

vorzugsweise

1.0 µg/Patient/Tag - 500 µg/Patient/Tag

Außerdem betrifft die Erfindung die Verwendung der Verbindungen gemäß Formel I zur Herstellung von Arzneimitteln.

Die Herstellung der Verbindungen der allgemeinen Formel I erfolgt erfindungsgemäß dadurch, daß eine Verbindung der allgemeinen Formel II

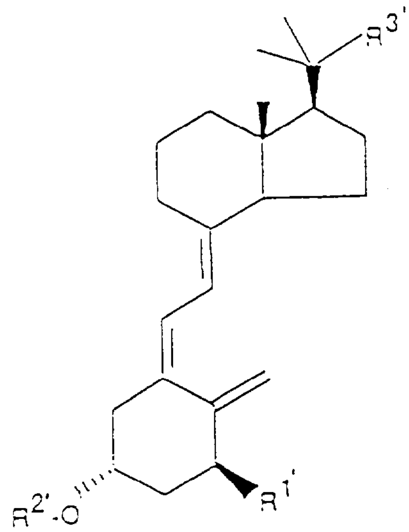

(II),

worin

$R^{1'}$ ein Wasserstoffatom oder eine geschützte Hydroxygruppe und
$R^{2'}$ eine alkalistabile Hydroxyschutzgruppe bedeuten sowie
$R^{3'}$ dieselbe Bedeutung wie $R^3$ in der letztendlich gewünschten Verbindung der allgemeinen Formel I hat, wobei gegebenenfalls vorhandene Hydroxygruppen geschützt sind,
durch Abspaltung der vorhandenen Hydroxyschutzgruppen und gegebenenfalls durch partielle oder vollständige Veresterung der Hydroxygruppe(n) in eine Verbindung der allgemeinen Formel I überführt wird.

Bei den alkalistabilen Hydroxyschutzgruppen, die im allgemeinen auch zum Schutz der Hydroxygruppen in 1-Position und/oder in der Seitenkette $R^{3'}$ dienen, handelt es sich vorzugsweise um die tert.-Butyldimethylsilyl-, die tert.-Butyldiphenylsilylgruppe oder um andere tertiäre Silylgruppen. Die Abspaltung der tertiären Silylgruppen gelingt z.B. unter Verwendung von Tetra-n-butyl-ammoniumfluorid.

Nach der Schutzgruppenabspaltung können freie Hydroxygruppen gewünschtenfalls verestert werden. Die Veresterung der verschiedenen freien Hydroxygruppen erfolgt nach gängigen Verfahren partiell aber vollständig mit dem entsprechenden Carbonsäurehalogenid (Halogenid=Chlorid, Bromid) oder Carbonsäureanhydrid.

Die Herstellung der erfindungsgemäßen Ausgangsverbindungen der allgemeinen Formel II geht aus von den bekannten Aldehyden der allgemeinen Formel III

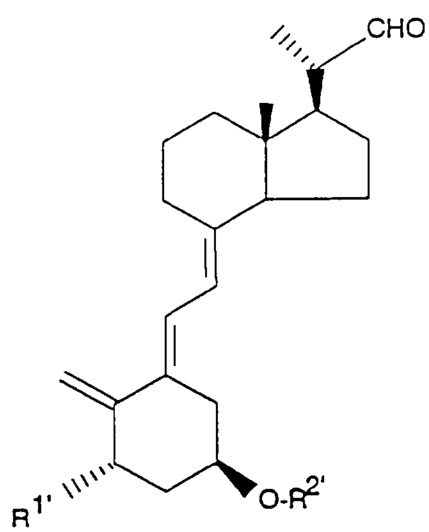

(III),

worin $R^{1'}$ und $R^{2'}$ die in der allgemeinen Formel II angegebene Bedeutung haben (M.J. Calverley, Tetrahedron 43, 4609, 1987; G. Neef et al., Tetrahedron Lett. 1991, 5073).

Deren $\alpha$-Alkylierung in üblicher Weise ergibt die dimethylierten Aldehyde der allgemeinen Formel IV, die anschließend in ebenfalls bekannter Weise durch triplett-sensibilisierte Photoisomerisierung in die zentralen Zwischenverbindungen der allgemeinen Formel V umgewandelt werden

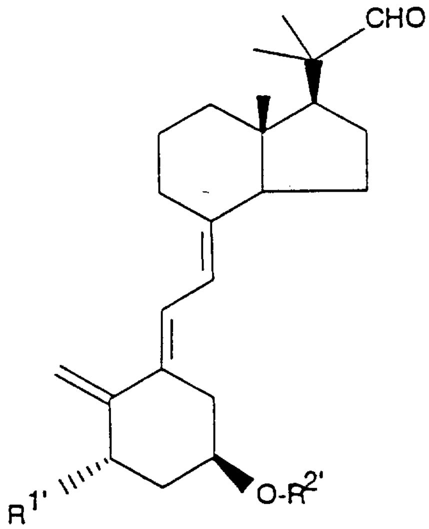
(IV)

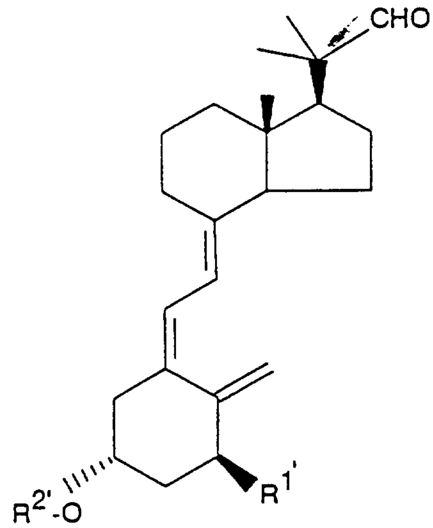
(V)

Die Methylierung wird z. B. mit Iodmethan oder Dimethylsulfat in Gegenwart einer Base (z.B. Alkalimetallhydroxide, -hydride, -amide) in einem aprotischen Lösungsmittel wie Tetrahydrofuran, Diethylether, Hexan, Ethylenglykoldimethylether oder Toluol, gegebenenfalls unter Zusatz eines Tetraalkylammoniumsalzes als Phasentransferkatalysator, durchgeführt.

Durch Bestrahlung mit ultraviolettem Licht in Gegenwart eines sogenannten "Triplettsensibilisators" (im Rahmen vorliegender Erfindung wird hierfür Anthracen verwendet) lassen sich die Verbindungen der allgemeinen Formel IV in die Verbindungen der allgemeinen Formel V überführen. Durch Spaltung der pi-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.

Anschließend muß noch der Rest $R^{3'}$ durch Kopplung eines Aldehyds der allgemeinen Formel V mit einem zur Kopplung geeigneten Vorläufer von $R^{3'}$ eingeführt werden. Dies geschieht in Analogie zu bekannten Verfahren; die experimentelle Durchführung dieser Verfahren findet sich beispielsweise bei M.J. Calverley, Tetrahedron 43, 4609, 1987; G. Neef und A. Steinmeyer, Tetrahedron Lett. 1991, 5073; int. Patentanmeldung WO 91/00855, DE-A 39 33 034 und DE-A 40 11 682. Als Beispiele seien genannt: Umsetzung des Aldehyds der allgemeinen Formel V mit einem Wittig-Reagens oder Reduktion des Aldehyds zum Alkohol und dessen Kettenverlängerung durch Umsetzung mit einer entsprechenden ω-Halogenverbindung.

Die nachfolgenden Beispiele dienen der näheren Erläuterung der vorliegenden Erfindung.

**Beispiel 1**

**1α,25-Dihydroxy-20,26,27-trimethyl-23-oxa-vitamin $D_3$**

a. Zu einer Suspension von 213 mg Natriumhydrid (80% in Öl) in 42 ml abs. THF tropft man unter Eiswasserkühlung eine Lösung von 4,5 g 1(S)-(tert.-Butyldimethylsilyloxy)-3(R)-(tert.-butyldiphenylsilyloxy)-20(S)-formyl-9,10-secopregna-5E,7E,10(19)-trien in 40 ml abs. THF. Nach Zugabe von 1,18 ml Iodmethan rührt man 2 h bei Raumtemperatur, gießt danach in Wasser und extrahiert mit Ethylacetat.

Das nach dem Einengen erhaltene Rohprodukt wird in 400 ml Toluol aufgenommen und nach Zusatz von 432 mg Anthracen und 0,2 ml Triethylamin 20 min bei Raumtemperatur in einer Rauchapparatur (Pyrex-Glas) mit einer Quecksilber-Hochdrucklampe (Philips HPK 125) bestrahlt. Nach dem Einengen der Reaktionslösung chromatographiert man den Rückstand an Kieselgel mit Hexan/Ethylacetat und erhält 2,38 g 1(S)-(tert.-Butyldimethylsilyloxy)-3(R)-(tert.-butyldiphenylsilyloxy)-20-formyl-20-methyl-9,10-secopregna-5Z,7E,10(19)trien als farbloses Öl.

$^1H$-NMR (CD $Cl_3$, 300MHz): δ = 0,52 ppm (s,3H,H-18); 4,23 (m,1H,H-3); 4,46 (m,1H,H-1); 4,85 u. 5,21(m, je1H,H-19); 6,04 u. 6,11 (d,J=11Hz,je1H,H-6 u. H-7); 9,66(s,1H,CHO).

b. Zur Lösung von 2,35 g des unter a. erhaltenen Aldehyds in 25 ml THF und 25 ml Methanol tropft man zunächst eine Lösung von 1,41 g $CeCl_3$ (Heptahydrat) in 25 ml Methanol. Nach Zugabe von 91 mg Natriumborhydrid rührt man 90 min bei 25°C, gießt anschließend in Wasser und extrahiert mit Ethylacetat. Chromatographie an Kieselgel

mit Hexan/Ethylacetat ergibt 1,86 g 1(S)-(tert.-Butyldimethylsilyloxy)-3(R)(tert.-butyl diphenylsilyloxy)-20-hydroxymethyl-20-methyl-9,10-secopregna-5Z,7E,10(19)-trien als farbloses Öl.

c. Ein Zweiphasensystem, bestehend aus 10,1 ml 25-proz. NaOH, 2,74 ml Bromessigsäure-tert.-butylester, 1,67 g des unter b. erhaltenen Alkohols in 25 ml Toluol und 48 mg Tetrabutylammoniumhydrogensulfat wird 6 h bei 50-60°C gerührt. Nach dem Abkühlen verdünnt man mit Toluol, trennt die Toluolphase ab, wäscht diese mit Wasser, trocknet über $Na_2SO_4$ und engt ein. Nach Chromatographie an Kieselgel mit Hexan/Ethylacetat erhält man 830 mg 1(S)-(tert.-Butyldimethylsilyloxy)-3(R)-(tert.-butyldiphenylsilyloxy)-20-(tert.-butoxycarbonylmethoxymethyl)-20-methyl-9,10-secopregna-5Z,7E,10(19)-trien-als gelbliches Öl.

d. Aus 490 mg Magnesium (Späne) und 1,5 ml Bromethan in 13 ml abs. THF stellt man in üblicher Weise die magnesiumorganische Verbindung her. Nach tropfenweiser Zugabe von 810 mg des unter c. erhaltenen tert.-Butylesters rührt man 3 h bei Raumtemperatur. Die Reaktionslösung wird zur Aufarbeitung in $NH_4Cl$-Lösung gegossen und mit Ethylacetat extrahiert.

e. Das nach dem Einengen erhaltene ölige Rohprodukt wird in 15 ml THF gelöst und nach Zugabe von 1,3 g Tetrabutylammoniumfluorid 2 h bei 50°C gerührt. Nach üblicher Aufarbeitung chromatographiert man an neutralem Aluminiumoxid mit Hexan/Ethylacetat. Durch Kristallisation der Hauptfraktion aus Diisopropylether/Ethylacetat erhält man 145 mg der Titelverbindung vom Smp. 146-148°C.

$^1$H-NMR ($CDCl_3$, 300 MHz): $\delta$=0,63 ppm (s,3H); 0,92 (s,3H); 1,00 (s,3H); 3,16 (s,2H); 3,23 (AB-q,J=9 u. 7 Hz, 2H); 4,23 (m,1H); 4,43 (m,1H); 4,98 (m,1H); 5,32 (m,1H); 5,90(d,J=11Hz,1H); 6,38 (d,J=11 Hz,1H).

**Beispiel 2**

**1(S),3(R)-Dihydroxy-20-(5-hydroxy-5-methyl-hexa-1E,3E-dien-1-yl)-20-methyl-9,10-secopregna-5Z,7E,10(19)-trien**

Die in der PCT-Anmeldung WO 91/00855 beschriebene Reaktionssequenz wurde mit 2,12 g des unter Beispiel 1a. erhaltenen Aldehyds durchgeführt. Nach Wittig-Reaktion mit Methoxycarbonyl-triphenylphosphoran, Reduktion mit Diisobutylaluminiumhydrid, Oxidation mit Pyridiniumdichromat, erneuter Wittig-Olefinierung mit Methoxycarbonyltriphenylphosphoran, Umsetzung des erhaltenen Esters mit Methyllithium und Schutzgruppenabspaltung mit Tetrabutylammoniumfluorid wurden 600 mg der Titelverbindung als farbloses Öl erhalten.
$[\alpha]_D$-65,5°($CDCl_3$,c = 0,525).
$^1$H-NMR($CDCl_3$,300 MHz) : $\delta$ = 0,57 ppm (s,3H); 1,04 (s,3H); 1,09 (s,3H); 1,34 (s,6H); 4,23 (m,1H); 4,43 (m,1H); 4,98 (m,1H); 5,32 (m,1H); 5,72 (d,J = 15 Hz, 1H); 5,87 (d,J = 10 Hz, 1H); 5,88 (dd, J = 15 u. 10 Hz, 1H); 6,00 (d,J = 11 Hz, 1H); 6,19 (dd,J = 15 u. 10 Hz, 1H); 6,37 (d,J = 11 Hz, 1H).

**Beispiel 3**

(5Z,7E)-(1S,3R)-20-Hydroxymethyl-20-methyl-9,10-seco-pregna-5,7,10(19)-trien-1,3-diol

Durch Silyletherspaltung des unter Beispiel 1b. erhaltenen Alkohols unter den Bedingungen des Beispiels 1e. erhält man die Titelverbindung vom Schmp. 183-185°C. $^1$H-NMR ($CDCl_3$ + DMSO-$d_6$, 300 MHz): $\delta$ = 0.22, 0.46 und 0.58 ppm (3 x s,je 3H, H-18 u. 20-methyl); 3.73 (m,1H,H-3); 3.95 (m,1H,H-1); 4.49 und 4.90 (2 x s,je 1H,H-19); 5.62 und 5.87 (2 x d,J = 11 Hz,je 1H,H-6 und H-7).

**Beispiel 4**

(5Z,7E)-(1S,3R)-20-methyl-20-vinyl-9,10-seco-pregna-5,7,10 (19)-trien-1,3-diol

Durch Umsetzung des unter Beispiel 1a. beschriebenen Aldehyds mit Methylentriphenylphosphoran und anschließende Silyletherspaltung gemäß Beispiel 1e. erhält man die Titelverbindung vom Schmp. 139-142°C, $[\alpha]_D$ -23.9° ($CHCl_3$, c = 0,255). $^1$H-NMR ($CDCl_3$, 300 MHz): $\delta$ = 0.57 ppm (s,3H,H-18); 1.03 u. 1.08 (2 x s,je 3H,20-methyl); 4.22 (m,1H,H-3); 4.43 (m,1H,H-1); 4.82 - 4.93 (m,2H,vinyl-$CH_2$); 4.99 u. 5.32 (2 x s,je 1H,H-19); 5.93 - 6.05 (m,2H,H-6 und vinyl-CH); 6.37 (d,J = 11 Hz,1H,H-7).

**Beispiel 5**

(5Z,7E)-(1S,3R)-20-Ethyl-20-methyl-9,10-seco-pregna-5,7,10 (19)-trien-1,3-diol

Durch Homologisierung des unter 1a. erhaltenen Aldehyds (z.B. Gemäß M.J. Calverley, Synlett 1990, 155), anschließende Reduktion gemäß Beispiel 1b., Überführung des so erhaltenen Alkohols in das entsprechende Iodid (z. B. nach G.L. Lange und C. Gottardo, Synth.Commun. 1990, 20, 1473), Reduktion des Iodids mit $LiAlH_4$ in THF und nachfolgend Silyletherspaltung erhält man die Titelverbindung. $^1$H-NMR ($CDCl_3$, 300 MHz): $\delta = 0.64$ ppm (s,3H,H-18); 0.87 u. 0.93 (2 x s,je 3H,20-methyl); 4.23 (m,1H,H-3); 4.42 (m,1H,H-1); 5.01 u. 5.34 (2 x s,je 1H,H-19); 6.01 u. 6.39 (2 x d,J = 11 Hz,je 1H,H-6 u. H-7).

**Beispiel 6**

1α,25-Dihydroxy-20-methyl-23-dehydro-vitamin $D_3$

Durch Homologisierung des unter 1a. beschriebenen Aldehyds (z.B. gemäß Synlett 1990,155), Wittig-Horner-Olefinierung des so erhaltenen homologen Aldehyds mit Dimethylphosphonoessigsäuremethylester (NaH, THF), Umsetzung des so erhaltenen ungesättigten Esters mit Methylmagnesiumbromid in THF und Silyletherspaltung erhält man die Titelverbindung als farbloses Öl. $^1$H-NMR ($CDCl_3$, 300 MHz): $\delta = 0.64$ ppm (s,3H,H-18); 0.89 u. 0.95 (2 x s, je 3H,20-methyl); 1.33 (s,6H,25-methyl); 4.23 (m,1H,H-3); 4.43 (m,1H,H-1); 5.00 u. 5.33 (2 x s,je 1H,H-19); 5.55 - 5.72 (m,2H,H-23 u. H-24); 6.00 u. 6.38 (2 x d,J = 11 Hz,je 1H,H-6 u. H-7).

**Beispiel 7**

1α,25-Dihydroxy-20-methyl-24-oxo-vitamin $D_3$

Durch Homologisierung des unter 1a. beschriebenen Aldehyds (z.B. Synlett 1990, 155), Wittig-Homer-Olefinierung mit Diethylphosphono-ethoxyessigsäure ethylester (nach W. Grell und H. Machleidt, Liebigs Ann.Chem. 699, 53, 1966), Addition von Methylmagnesiumbromid, Enoletherspaltung (70-proz. Essigsäure) und Entfemung der Silyletherschutzgruppen erhält man die Titelverbindung vom Schmp. 141-144°C, $[\alpha]_D$ +14,7° ($CHCl_3$, c = 0.505). $^1$H-NMR ($CDCl_3$, 300 MHz): $\delta = 0.65$ ppm (s,3H,H-18); 0.90 u. 0.98 (2 x s,je 3H,20-methyl); 1.40 (s,6H,25-methyl); 4.23 (m,1H,H-3); 4.44 (m,1H,H-1); 5.00 u. 5.33 (2 x breites s,je 1H,H-19); 6.01 u. 6.38 (2 x d,J = 11 Hz,je 1 H,H-6 u. H-7).

**Patentansprüche**

**1.** 20-Methyl-substituierte Vitamin D-Derivate der allgemeinen Formel I

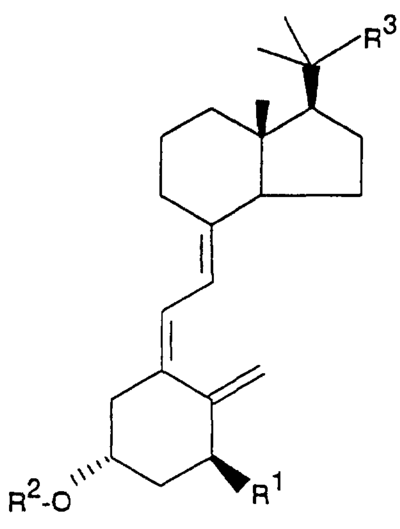

(I),

worin

$R^1$ ein Wasserstoffatom, eine Hydroxygruppe oder eine Alkanoyloxygruppe mit 1 bis 12 Kohlenstoffatomen

bzw. eine Benzoyloxygruppe,
$R^2$ ein Wasserstoffatom oder eine Alkanoylgruppe mit 1 bis 12 Kohlenstoffatomen bzw. eine Benzoylgruppe und
$R^3$ einen gesättigten oder ungesättigten, geradkettigen oder verzweigten Kohlenwasserstoffrest mit bis zu 18 C-Atomen, der gegebenenfalls durch eine oder mehrere $C_{3-10}$-Cycloalkyl- oder Cycloalkenylrest(e), letztere mit bis zu 2 Doppelbindungen unterbrochen und/oder substituiert ist, der gegebenenfalls mit einer oder mehreren Hydroxy-, Oxo-, Aminogruppe(n) und/oder einem oder mehreren Halogenatom(en) substituiert sowie gegebenenfalls ein oder mehrere Sauerstoff-, Schwefel- und/oder >NH-Gruppe(n) als verbrückende(s) Kettenglied(er) im Kohlenwasserstoffrest aufweist,
bedeuten.

**2.** Vitamin D-Derivate nach Anspruch 1, dadurch gekennzeichnet, daß $R^3$ einer der folgenden Reste ist:

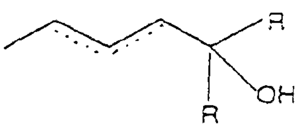

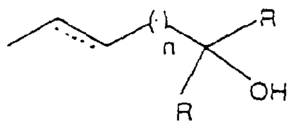

n = 1 - 3

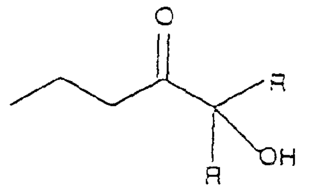

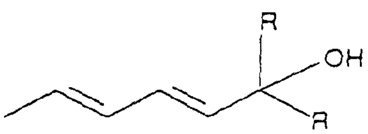

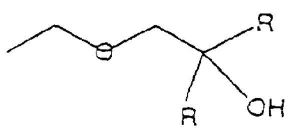

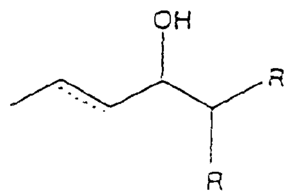

R = $C_1$-$C_4$-Alkyl, -Hydroxyalkyl, -O-Alkyl.

**3.** 1α,25-Dihydroxy-20,26,27-trimethyl-23-oxa-vitamin $D_3$,

1(S),3(R)-Dihydroxy-20-(5-hydroxy-5-methyl-hexa-1E,3E-dien-1-yl)-20-methyl-9,10-secopregna-5Z,7E,10(19)-trien,
1α,25-Dihydroxy-20-methyl-vitamin $D_3$,
1α,25-Dihydroxy-20-methyl-24-homo-vitamin $D_3$,
1α,24(S)-Dihydroxy-20-methyl-vitamin $D_3$,
1α,25-Dihydroxy-20-methyl-23-oxa-vitamin $D_3$,
1α,24(R),25-Trihydroxy-20-methyl-vitamin $D_3$,
1α,24(S),25-Trihydroxy-20-methyl-vitamin $D_3$,
1α,25-Dihydroxy-20-methyl-24-oxo-vitamin $D_3$,
(5Z,7E)-(1S,3R)-20-Methyl-20-vinyl-9,10-seco-pregna-5,7,10(19)-trien-1,3-diol,
(5Z,7E)-(1S,3R)-20-Ethyl-20-methyl-9,10-seco-pregna-5,7,10(19)-trien-1,3-diol,
(5Z,7E)-(1S,3R)-20-Hydroxymethyl-20-methyl-9,10-seco-pregna-5,7,10(19)-trien-1,3-diol,
1α,25-Dihydroxy-20-methyl-23,24-dehydro-vitamin $D_3$,
1α,25-Dihydroxy-20,26,27-trimethyl-23,24-dehydro-vitamin $D_3$.

**4.** Verfahren zur Herstellung von 20-Methyl-substituierten Vitamin D-Derivaten der allgemeinen Formel I nach mindestens einem der Ansprüche 1-3,
dadurch gekennzeichnet, daß eine Verbindung der allgemeinen Formel II

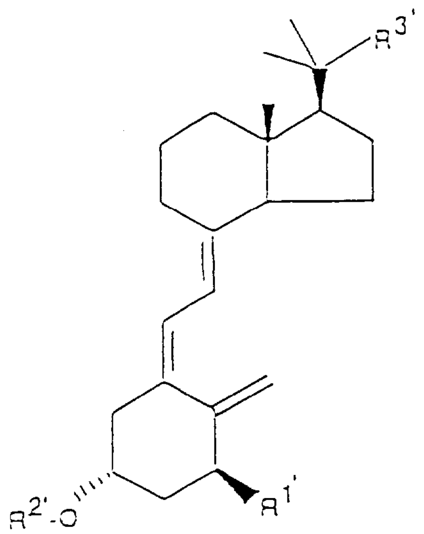

(II),

worin

$R^{1'}$ ein Wasserstoffatom oder eine geschützte Hydroxygruppe und
$R^{2'}$ eine alkalistabile Hydroxyschutzgruppe bedeuten sowie
$R^{3'}$ dieselbe Bedeutung wie $R^3$ in der leztendlich gewünschten Verbindung der allgemeinen Formel I hat, wobei gegebenenfalls vorhandene Hydroxygruppen geschützt sind,
durch Abspaltung der vorhandenen Hydroxyschutzgruppen und gegebenenfalls durch partielle oder vollständige Veresterung der Hydroxygruppe(n) in eine Verbindung der allgemeinen Formel I überführt wird.

**5.** Zwischenverbindungen der allgemeinen Formel V

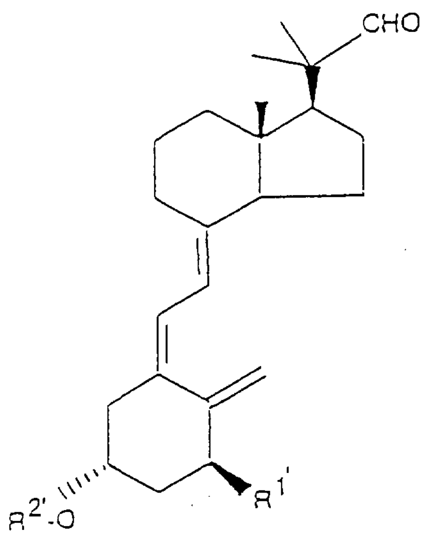

(V)

worin
$R^{1'}$ und $R^{2'}$ die in Anspruch 4 definierte Bedeutungen haben, mit der maßgabe, daß Formel (V) verschieden von Formel (I) gemäß Anspruch 1 ist.

**6.** Pharmazeutische Präparate, dadurch gekennzeichnet, daß sie mindestens eine Verbindung gemäß den Ansprüchen 1 bis 3 sowie einen pharmazeutisch verträglichen Träger enthalten.

**7.** Verwendung der Verbindungen gemäß den Ansprüchen 1 bis 3 zur Herstellung von Arzneimitteln.

**Claims**

1. 20-methyl-substituted vitamin D derivatives of the general formula I

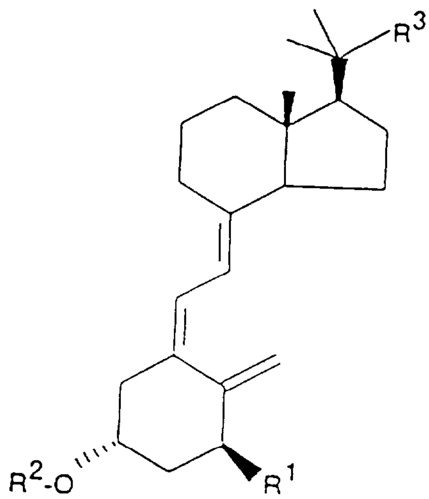

(I),

wherein

$R^1$ is a hydrogen atom, a hydroxy group or an alkanoyloxy group having from 1 to 12 carbon atoms, or a benzoyloxy group,
$R^2$ is a hydrogen atom or an alkanoyl group having from 1 to 12 carbon atoms, or a benzoyl group and
$R^3$ is a saturated or unsaturated, straight-chained or branched hydrocarbon radical having up to 18 carbon atoms that is optionally interrupted and/or substituted by one or more $C_{3-10}$-cycloalkyl or -cycloalkenyl radical(s), the latter having up to 2 double bonds, and
that is optionally substituted by one or more hydroxy, oxo and/or amino group(s) and/or one or more halogen atom(s) and optionally has one or more oxygen, sulfur and/or NH group(s) as bridging chain member(s) in the hydrocarbon radical.

2. Vitamin D derivatives according to claim 1, characterised in that $R^3$ is one of the following radicals:

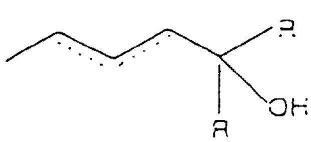

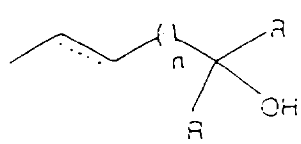

n = 1 - 3

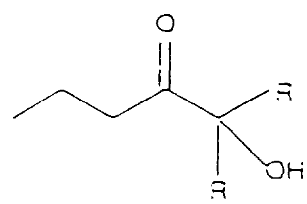

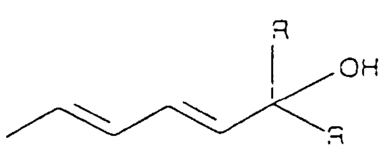

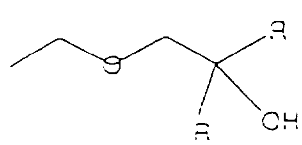

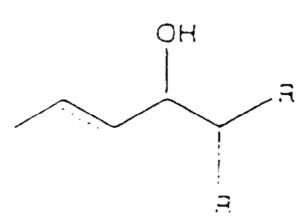

wherein
R = $C_1$-$C_4$-alkyl, -hydroxyalkyl or -O-alkyl.

3. 1α,25-Dihydroxy-20,26,27-trimethyl-23-oxa-vitamin $D_3$,

1(S),3(R)-dihydroxy-20-(5-hydroxy-5-methyl-hexa-1E,3E-dien-1-yl)-20-methyl-9,10-secopregna-5Z,7E,10(19)-triene,
1α,25-dihydroxy-20-methyl-vitamin $D_3$,
1α,25-dihydroxy-20-methyl-24-homo-vitamin $D_3$,
1α,24(S)-dihydroxy-20-methyl-vitamin $D_3$,
1α,25-dihydroxy-20-methyl-23-oxa-vitamin $D_3$,
1α,24(R),25-trihydroxy-20-methyl-vitamin $D_3$,
1α,24(S),25-trihydroxy-20-methyl-vitamin $D_3$,
1α,25-dihydroxy-20-methyl-24-oxo-vitamin $D_3$,
(5Z,7E)-(1S,3R)-20-methyl-20-vinyl-9,10-secopregna-5,7,10(19)-triene-1,3-diol,
(5Z,7E)-(1S,3R)-20-ethyl-20-methyl-9,10-secopregna-5,7,10(19)-triene-1,3-diol,
(5Z,7E)-(1S,3R)-20-hydroxymethyl-20-methyl-9,10-secopregna-5,7,10(19)-triene-1,3-diol,
1α,25-dihydroxy-20-methyl-23,24-dehydro-vitamin $D_3$ and
1α,25-dihydroxy-20,26,27-trimethyl-23,24-dehydro-vitamin $D_3$.

**4.** Process for the manufacture of 20-methyl-substituted vitamin D derivatives of the general formula I according to at least one of claims 1 to 3, characterised in that a compound of the general formula II

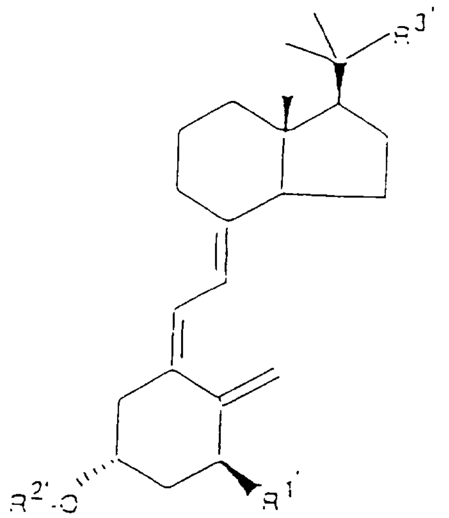

(II),

wherein

$R^{1'}$ is a hydrogen atom or a protected hydroxy group and
$R^{2'}$ is an alkali-stable hydroxy-protecting group and
$R^{3'}$ has the same meaning as $R^3$ in the ultimately desired compound of the general formula I, any hydroxy groups that may be present being protected,
is converted into a compound of the general formula I by removal of any hydroxy-protecting groups that are present and optionally by partial or complete esterification of the hydroxy group(s).

**5.** Intermediates of the general formula V

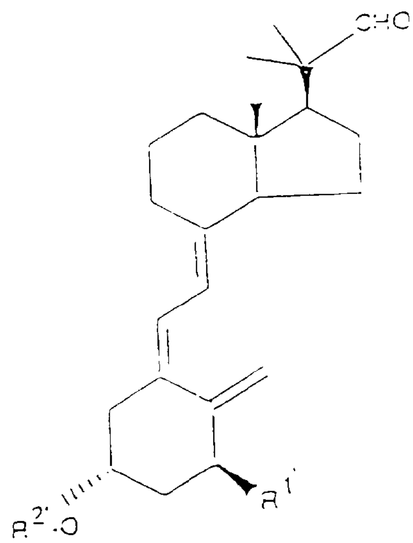

(V)

wherein

$R^{1'}$ and $R^{2'}$ have the meanings defined in claim 4, with the proviso that formula (V) is different from formula (I) according to claim 1.

6. Pharmaceutical preparations, characterised in that they contain at least one compound according to claims 1 to 3 and a pharmaceutically acceptable carrier.

7. Use of the compounds according to claims 1 to 3 in the manufacture of medicaments.

## Revendications

1. Dérivés de vitamine D à substitution 20-méthyle, de formule générale I

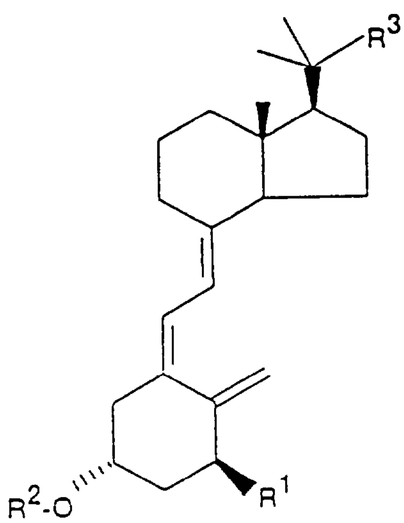

(I),

dans laquelle

$R^1$ est un atome d'hydrogène, un groupe hydroxy ou un groupe alcanoyloxy ayant de 1 à 12 atomes de carbone, ou un groupe benzoyloxy,
$R^2$ est un atome d'hydrogène ou un groupe alcanoyle ayant de 1 à 12 atomes de carbone ou un groupe benzoyle, et
$R^3$ est un résidu hydrocarboné à chaîne droite ou ramifiée, saturé ou insaturé, ayant jusqu'à 18 atomes de carbone, qui peut éventuellement être interrompu et/ou substitué par un ou plusieurs résidus cycloalkyle ou cycloalcényle en $C_{3-10}$, ces derniers ayant jusqu'à deux doubles liaisons, résidu qui éventuellement est substitué par un ou plusieurs groupes hydroxy, oxo, amino et/ou par un ou plusieurs hétéroatomes, et éventuellement comporte un ou plusieurs groupes oxygène, soufre et/ou >NH comme chaînons pontants dans le résidu hydrocarboné.

**2.** Dérivés de vitamine D selon la revendication 1, caractérisés en ce que $R^3$ est l'un des résidus suivants :

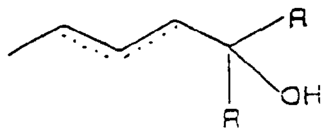

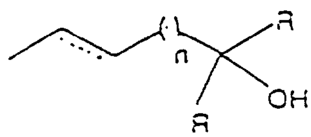

n = 1 - 3

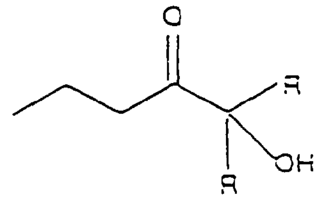

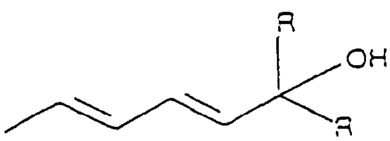

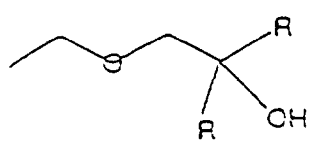

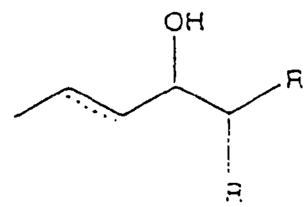

R est un radical alkyle, hydroxyalkyle ou O-alkyle en $C_1$-$C_4$.

**3.** 1α,25-dihydroxy-20,26,27-triméthyl-23-oxa-vitamine $D_3$,

1(S),3(R)-dihydroxy-20-(5-hydroxy-5-méthyl-hexa-1E,3E-diène-1-yl)-20-méthyl-9,10-sécopregna-5Z,7E,10-(19)-triène,
1α,25-dihydroxy-20-méthyl-vitamine $D_3$,
1α,25-dihydroxy-20-méthyl-24-homo-vitamine $D_3$,
1α,24(S)-dihydroxy-20-méthyl-vitamine $D_3$,
1α,25-dihydroxy-20-méthyl-23-oxa-vitamine $D_3$,
1α,24(R),25-trihydroxy-20-méthyl-vitamine $D_3$,
1α,24(S),25-trihydroxy-20-méthyl-vitamine $D_3$,
1α,25-dihydroxy-20-méthyl-24-oxo-vitamine $D_3$,
(5Z,7E)-(1S,3R)-20-méthyl-20-vinyl-9,10-sécopregna-5,7,10(19)-triène-1,3-diol,
(5Z,7E)-(1S,3R)-20-éthyl-20-méthyl-9,10-sécopregna-5,7,10(19)-triène-1,3-diol,
(5Z,7E)-(1S,3R)-20-hydroxyméthyl-20-méthyl-9,10-séco-pregna-5,7,10(19)-triène-1,3-diol,
1α,25-dihydroxy-20-méthyl-23,24-déhydro-vitamine $D_3$,
1α,25-dihydroxy-20,26,27-triméthyl-23,24-déhydro-vitamine $D_3$.

**4.** Procédé pour préparer des dérivés de vitamine D à substitution 20-méthyle de formule générale I selon au moins l'une des revendications 1 à 3, caractérisé en ce qu'on convertit un composé de formule générale II

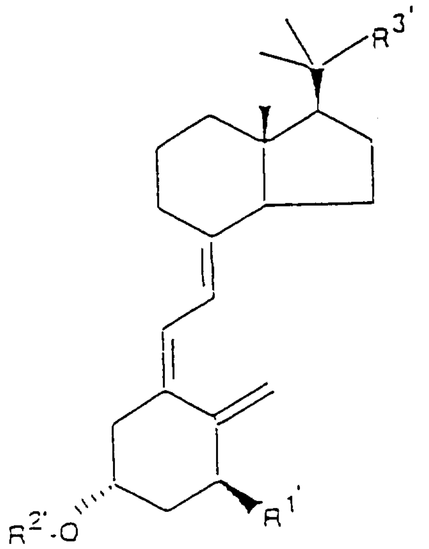

(II),

dans laquelle

$R^{1'}$ est un atome d'hydrogène ou un hydroxy protégé, et
$R^{2'}$ est un groupe hydroxy-protecteur stable vis-à-vis des alcalis, et
$R^{3'}$ a la même signification que $R^3$ dans le composé finalement souhaité de formule générale I, auquel cas les groupes hydroxy éventuellement présents sont protégés,
par élimination des groupes hydroxy-protecteurs présents et éventuellement par estérification partielle ou totale du ou des groupes hydroxy, pour donner un composé de formule générale I.

**5.** Composés intermédiaires de formule générale V

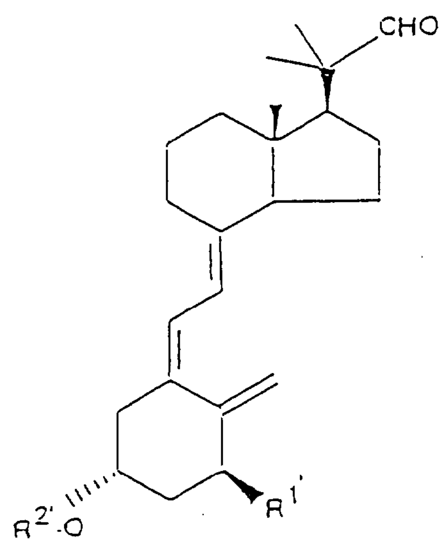

(V)

dans laquelle
$R^{1'}$ et $R^{2'}$ ont les significations données dans la revendication 4, du moment que la formule (V) est différente de la formule (I) selon la revendication 1.

**6.** Préparations pharmaceutiques, caractérisées en ce qu'elles contiennent au moins un composé selon les revendications 1 à 3 ainsi qu'un excipient acceptable d'un point de vue pharmaceutique.

**7.** Utilisation des composés selon les revendications 1 à 3 pour préparer des médicaments.